Europäisches Patentamt

European Patent Office

Office européen des brevets

(11) Veröffentlichungsnummer: **0 026 478**
**A2**

(12) # EUROPÄISCHE PATENTANMELDUNG

(21) Anmeldenummer: 80105828.0

(22) Anmeldetag: 25.09.80

(51) Int. Cl.³: **A 61 N 1/36**
**G 06 F 13/00, G 06 F 15/42**

(30) Priorität: 27.09.79 DE 2939197

(43) Veröffentlichungstag der Anmeldung:
08.04.81 Patentblatt 81/14

(84) Benannte Vertragsstaaten:
CH DE FR GB IT LI NL SE

(71) Anmelder: SIEMENS AKTIENGESELLSCHAFT Berlin
und München
Postfach 22 02 61
D-8000 München 22(DE)

(72) Erfinder: Ekwall, Christer
Jungfrudansen 9
S-171 56 Solna(SE)

(72) Erfinder: Elmqvist, Häkan, Dr.
Sunnderdahlsvägen 7
S-161 38 Bromma(SE)

(54) Signalverarbeitungsvorrichtung, insbesondere für Herzschrittmacher.

(57) Die Erfindung bezieht sich auf eine Signalverarbeitungsvorrichtung, insbesondere für Herzschrittmacher, mit einem Signalempfangsteil (1 bis 9) und einer Zuleiteinrichtung (10 bis 19), die bestimmte Signalinformationen dafür vorgesehenen Informationsspeichern zur Speicherung zuleitet. Ziel der Erfindung ist es, eine solche Signalverarbeitungsvorrichtung zu schaffen, die bei geringstmöglichem Schaltungsaufwand eine unerwünschte Umprogrammierung von Informationsspeichern immer mit absoluter Sicherheit verhindert. Dieses Ziel wird erfindungsgemäß dadurch erreicht, daß einer aus einer Mehrzahl von Informationsspeichern (20 bis 26) aufgebauten Speicherbank ein Verschlußregister (27) zugeordnet ist, in das für jeden Informationsspeicher ein speziell diesem zugeordnetes Verschlußsignal einspeicherbar ist, aufgrund dessen eine Änderung des gespeicherten Informationsgehaltes so lange unmöglich gemacht wird, wie von außen nicht ein Öffnungssignal für einen betreffenden Informationsspeicher in das Verschlußregister eingegeben wird (Figur).

SIEMENS AKTIENGESELLSCHAFT    Unser Zeichen
Berlin und München           VPA 79 P 5958   EUR

Signalverarbeitungsvorrichtung, insbesondere für Herzschrittmacher

Die Erfindung bezieht sich auf eine Signalverarbeitungsvorrichtung, insbesondere für Herzschrittmacher, mit
einem Signalempfangsteil und einer Zuleiteinrichtung,
die bestimmte Signalinformationen dafür vorgesehenen
Informationsspeichern zur Speicherung zuleitet.

Signalverarbeitungsvorrichtungen dieser Art sollen insbesondere für den Einsatz bei Herzschrittmachern eine
Anzahl von Informationsspeichern aufweisen, die einzeln
mit Signalinformationen, wie Frequenz, Amplitudenverlauf, Hysterese usw., speicherbar sind. Generell erfolgt
diese Speicherung in der Weise, daß dem implantierten
Herzschrittmacher mittels geeignetem Programmgeber von
außen, d. h. durch die Haut hindurch, alle nötigen Informationen über das zu verwendende Schrittmachersignal
zur Zuleitung in die Informationsspeicher eingegeben
werden. In der Praxis sieht dies so aus, daß alle neuen
Informationen durch den Arzt vorgegeben und durch diesen

Kue 5 Rl / 24.9.1979

schließlich auch programmäßig eingegeben werden. Der Patient selbst kann dann im Normalfall keinen Einfluß auf die eingegebenen Informationen ausüben. Entgegen dieser üblichen Anwendung besteht jedoch oft der Wunsch, daß wenigstens bei ganz bestimmten Informationen in begrenztem Maße eine nachträgliche Regulierung der Information auch durch den Patienten möglich sein soll. Beispielsweise sollte es möglich sein, daß eine spezielle Information den unterschiedlichen Anforderungen des Tagesrhythmus entsprechend in gewissen Grenzen durch den Patienten veränderbar sein soll. Die Vorsehung einer solchen Veränderungsmöglichkeit durch den Patienten setzt jedoch voraus, daß Maßnahmen getroffen werden, die dafür sorgen, daß durch den Patienten auch nur die zugelassene Größe, nicht jedoch andere bereits eingespeicherte Information, verändert werden kann.

Aufgabe vorliegender Erfindung ist es, eine Signalverarbeitungsvorrichtung, insbesondere zur Anwendung bei Herzschrittmachern, zu schaffen, die bei geringstmöglichem Schaltungsaufwand eine unerwünschte Umprogrammierung immer mit absoluter Sicherheit verhindert.

Die Aufgabe wird erfindungsgemäß dadurch gelöst, daß einer aus einer Mehrzahl von Informationsspeichern aufgebauten Speicherbank ein Verschlußregister zugeordnet ist, in das für jeden Informationsspeicher ein speziell diesem zugeordnetes Verschlußsignal einspeicherbar ist, aufgrund dessen eine Änderung des gespeicherten Informationsgehaltes so lange unmöglich gemacht wird, wie von außen nicht ein Öffnungssignal für einen betreffenden Informationsspeicher in das Verschlußregister eingegeben wird.

Weitere Vorteile und Einzelheiten der Erfindung ergeben sich aus der nachfolgenden Beschreibung eines Ausführungsbeispieles anhand der Zeichnung in Verbindung mit den Unteransprüchen.

Die Figur zeigt die Erfindung im Prinzipschaltbild.

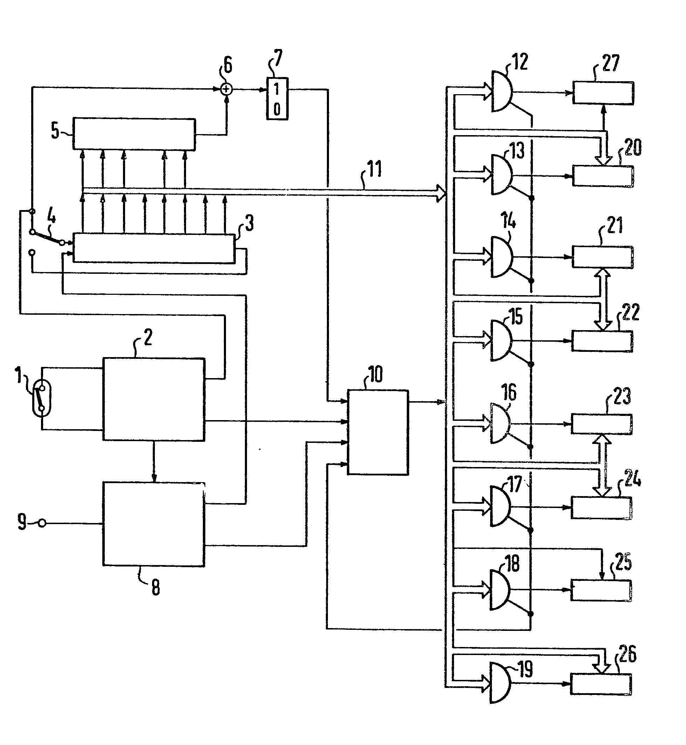

In der Fig. 1 ist mit 1 ein Einschalter (Reed-Kontakt) zur Eingabe von Signalinformationen in einen Signalempfangsteil 2 bis 9 eines implantierbaren Herzschrittmachers bezeichnet. Der Schalter 1 ist von außen, d. h. durch die Haut des Schrittmacherpatienten hindurch, durch Magnet betätigbar. Der Signalempfangsteil umfaßt ein Informationserkennungteil 2, einen Informationszwischenspeicher 3 (Shiftregister) mit Umschalter 4 sowie ein Kontrollregister 5 mit Vergleichsglied 6 und Fehlererkennungsglied 7. Ein zentraler Taktgeber ist mit 8 bezeichnet. Er wird an einem Eingang 9 mit Grundtaktimpulsen beaufschlagt. Die vom Informationszwischenspeicher 3 erfaßten Daten werden, sofern die Fehlerkontrolle keinen Fehler ergeben hat, gesteuert von einer Steuerlogik 10 über eine Informationsleitung 11 und über Adressendekoder 12 bis 19 einer Bank aus Informationsspeichern 20 bis 26 zugeleitet. Die einzelnen Informationsspeicher sind so aufgebaut und werden so angesteuert, daß jeder Speicher separat eine spezielle dem Herzschrittmachersignal zugeordnete Information trägt. Der Informationsspeicher 20 trägt so z. B. die Information über die Frequenz der Herzschrittmacherimpulse, während der Informationsspeicher 22 die Information über Impulsdauer und der Informationsspeicher 23 die Information über Hysterese trägt. Der Informationsspeicher 24 beinhaltet die Amplitudeninformation, während die Speicher 25 und 26 z. B. ausnutzbar sind zu Speicherungen von Informationen, die die Reizschwelle des Herzens betreffen und die z. B. nach dem Varioprinzip (schrittweise Absenkung der Herzschrittmacheramplitude bis Erreichen der Reizschwelle) gewonnen werden. Der Informationsspeicher 21 kann als Reservespeicher dienen.

Erfindungswesentlich ist nun, daß sämtlichen dieser Speicher ein Verschlußregister 27 zugeordnet ist. Dieses Verschlußregister 27 ist von außen kontrollierbar und er beinhaltet Verschlußinformationen, aufgrund derer eine Änderung des gespeicherten Informationsgehaltes für jeden einzelnen Informationsspeicher 20 bis 26 so lange unmöglich gemacht wird, wie von außen nicht ein Öffnungssignal für einen betreffenden Informationsspeicher in das Verschlußregister eingegeben wird. Im vorliegenden Ausführungsbeispiel handelt es sich bei dem Verschlußregister um ein solches, das für jeden einzelnen Speicher 20 bis 26 eine 2-Bit-Information beinhaltet. Sofern das Register 27 in der speziell einem Informationsspeicher 20 bis 26 zugeordneten Registerstelle eine Nein-Information trägt, ist auch der zugehörige Informationsspeicher für jedwede Änderung der gespeicherten Information unzugänglich. Befindet sich jedoch die entsprechende Registerstelle in einer Ja-Formation, so besteht die Möglichkeit einer Änderung einer eingespeicherten Information in dem betreffenden Informationsspeicher durch Einwirkung von außen.

In der Praxis ist es so, daß gewöhnlich sämtliche Informationen für die einzelnen Informationsspeicher 20 bis 26 durch den Arzt eingegeben werden. In einigen speziellen Fällen wird keine Notwendigkeit nach einer Änderung der einmal eingegebenen Information zumindest für einen gewissen Zeitraum bestehen. Der Arzt kann demnach mit Eingabe der Informationen gleichzeitig auch in das Verschlußregister 27 ein Verschließsignal für sämtliche Informationen der einzelnen Speicher eingeben. In anderen Fällen wird es so sein, daß dem Bedarf des Patienten entsprechend (z. B. zur Anpassung einzelner Informationen an den geänderten Tagesrhythmus) dann und wann Informationskorrektur notwendig und erwünscht ist. Damit eine solche Informationskorrektur durch den Patienten selbst vorgenommen werden kann, wird der Arzt durch Eingabe eines speziellen Öffnungssignales in das Verschlußregister 27 eine Variationsmöglichkeit für den Patienten speziell nur für diese Information in diesem speziellen Informationsspeicher zulassen. Für sämtliche anderen Informationsspeicher, in denen die einmal eingegebene Information unveränderbar sein soll, bleibt das einmal eingegebene Verschlußsignal. Ein Beispiel für recht häufige Anwendung ist jenes, daß die Schrittmacherfrequenz über den Tagesablauf veränderbar sein soll. In diesem Fall wird dann also der Arzt für alle Informationsspeicher, außer dem Informationsspeicher 20 für die Frequenz, im Verschlußregister 27 ein Verschlußsignal vorsehen. Lediglich für den Informationsspeicher 20 wird im Verschlußregister ein Öffnungsignal einprogrammiert. Aufgrund dieses Öffnungssignales kann dann der Patient jederzeit im Laufe des Tages eine am geänderten Bedarf angepaßte Frequenzkorrektur vornehmen. Damit ist also bei geringstmöglichem Schaltungsaufwand eine Möglichkeit geschaffen, wie Umprogrammierungen erwünschter Art leicht vorgenommen, solche von unerwünschter Art hingegen gleichzeitig immer mit absoluter Sicherheit verhindert werden.

Patentansprüche

1. Signalverarbeitungsvorrichtung, insbesondere für Herzschrittmacher, mit einem Signalempfangsteil und einer Zuleiteinrichtung, die bestimmte Signalinformationen dafür vorgesehenen Informationsspeichern zur Speicherung zuleitet, d a d u r c h   g e k e n n - z e i c h n e t ,  daß einer aus einer Mehrzahl von Informationsspeichern (20 bis 26) aufgebauten Speicherbank ein Verschlußregister (27) zugeordnet ist, in das für jeden Informationsspeicher ein speziell diesem zugeordnetes Verschlußsignal einspeicherbar ist, aufgrund dessen eine Änderung des gespeicherten Informationsgehaltes so lange unmöglich gemacht wird, wie von außen nicht ein Öffnungssignal für einen betreffenden Informationsspeicher in das Verschlußregister (27) eingegeben wird.

2. Signalverarbeitungsvorrichtung nach Anspruch 1, d a d u r c h   g e k e n n z e i c h n e t ,  daß das Verschlußregister (27) den einzelnen Informationsspeichern (20 bis 26) separat zugeordnete Registerstellen für Verschließ- oder Öffnungssignale beinhaltet.

3. Signalverarbeitungsvorrichtung nach Anspruch 1, d a d u r c h   g e k e n n z e i c h n e t ,  daß die Informationen 2-Bit-Informationen sind, von denen die Nein-Information dem Verschließsignal und die Ja-Information dem Öffnungssignal entspricht.

4. Signalverarbeitungsvorrichtung nach einem der Ansprüche 1 bis 3, d a d u r c h   g e k e n n - z e i c h n e t ,  daß ihr Programmiergeräte zugeordnet sind, von denen eines für spezielle Bedienung, z. B. durch den Arzt, eine beliebige Programmierung

**0026478**

der einzelnen Informationsspeicher (20 bis 26) bei gleichzeitiger Eingabe erwünschter Informationssignale für das Verschlußregister (27) ermöglicht.

5. Signalverarbeitungsvorrichtung nach Anspruch 4, d a d u r c h   g e k e n n z e i c h n e t , daß ein weiteres Programmiergerät so ausgestaltet ist, daß es eine Umprogrammierung des Verschlußregisters (27) nur in beschränktem Maße auf Öffnungssignale für bestimmte einzelne Informationsspeicher, deren Umprogrammierung möglich sein soll, erlaubt.